# EUROPEAN PATENT APPLICATION

(11) **EP 2 246 019 A1**
(43) Date of publication of application: **03.11.2010**
(21) Application number: 09706528.8
(22) Date of filing: 28.01.2009
(51) Int. Cl.: A61F 13/15, A61F 13/472, A61F 13/511, A61F 13/56

(54) **ABSORBENT ARTICLE**

(30) Priority: 28.01.2008 JP 2008016469
(71) Applicant: Unicharm Corporation, Ehime 799-0111 (JP)
(72) Inventor: KUDO, Jun, . (JP)
(74) Representative: Murgatroyd, Susan Elizabeth
(86) International application number: PCT/JP2009/051307
(87) International publication number: WO 2009/096402

(57) **Abstract**

An absorbent article having a napkin body (2) comprising as the essentials a liquid-permeable top sheet (3), a liquid-impermeable back sheet (4), and a liquid-absorbing member (5) placed between the top sheet (3) and the back sheet (4), wherein a removable cover sheet (20A) having a length and a width which do not exceed respectively the length and width of the napkin body (2) is provided on the top sheet (3) side of the napkin body (2) in contact with the top sheet (3).

## Description

### TECHNICAL FIELD

The present invention relates to absorbent articles, such as a sanitary napkin, a liner, and an incontinence pad, which are pasted to undergarment for use.

### BACKGROUND ART

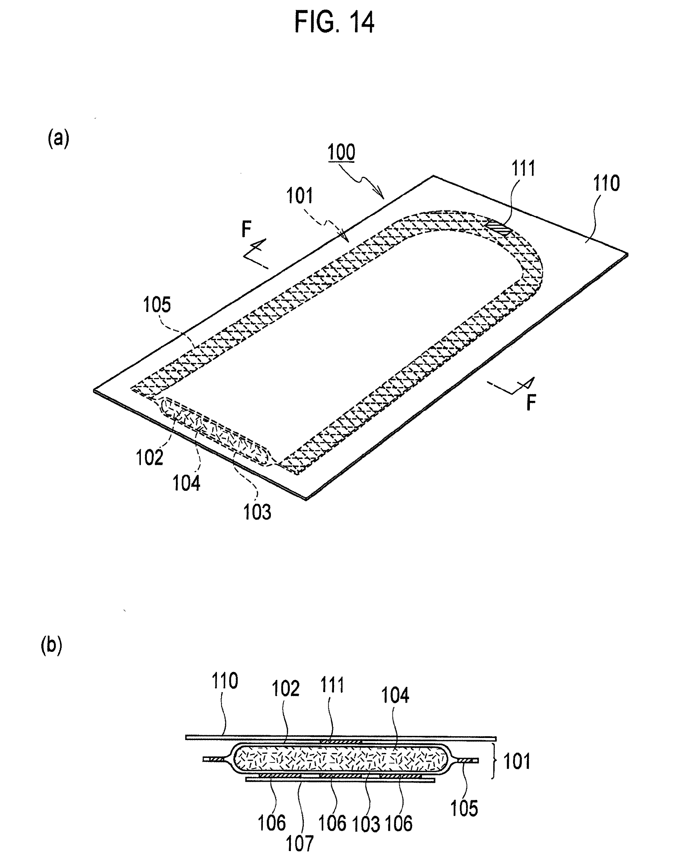

Patent Document 1 discloses a first conventional example of a sanitary napkin, which is an absorbent article. As shown in Figs. 14(a), and (b), the sanitary napkin 100 of this first conventional example includes an napkin main body 101, which is an absorbent main body, and a cover sheet 110 detachable from the napkin main body 101. The napkin main body 101 includes a liquid-permeable topsheet 102, a liquid-impermeable backsheet 103, and an absorber 104. The absorber 104 is disposed between the topsheet 102 and the backsheet 103, and configured to absorb liquid. Outer peripheries of the topsheet 102 and the backsheet 103 are bonded to each other by means of a hot-melt-bonded portion 105 (shown in Fig. 14(a) by the cross hatching). Adhesive portions 106 are provided onto an outer surface of the backsheet 103. A release sheet 107 is bonded to outer surfaces of the adhesive portions 106.

The cover sheet 110 is disposed on a surface of the topsheet 102. The cover sheet 110 is temporarily attached to the napkin main body 101 by means of a hot-melt-bonded portion 111 disposed on a portion of the topsheet 102.

Next, brief description is given of a mounting procedure of the sanitary napkin 100. First, the release sheet 107 is peeled off. Next, the napkin main body 101 with the cover sheet 110 attached thereto is positioned at an appropriate position of undergarment. The positioned napkin main body 101 with the cover sheet 110 attached thereto is pressed to the undergarment with the fingers or the palms. The napkin main body 101 with the cover sheet 110 attached thereto is pasted to the undergarment by means of the adhesive portions 106. Next, the cover sheet 110 is detached from the napkin main body 101 to complete the mounting.

Patent Document 2 discloses a second conventional example of a sanitary napkin. As shown in Figs. 15(a) and (b), a sanitary napkin 120 according to this second conventional example includes a napkin main body 121, which is an absorbent main body, and a cover sheet 130 detachable from the napkin main body 121. The napkin main body 121 includes a liquid-permeable topsheet 122, a liquid-impermeable backsheet 123, an absorber 124 configured to absorb liquid, and a pair of wings 125. The absorber 124 is disposed between the topsheet 122 and the backsheet 123. The pair of wings 125 project in width directions from outer edges of the topsheet 122 and the backsheet 123 extending in a longitudinal direction.

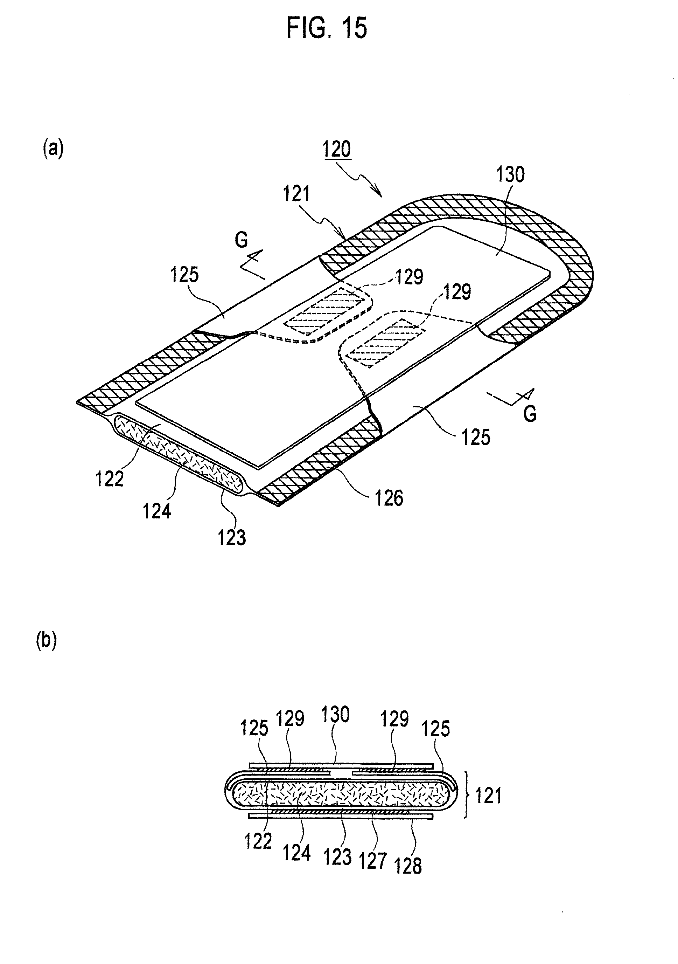

The outer edges of the topsheet 122 and the backsheet 123 are bonded to each other by means of a hot-melt-bonded portion 126 (shown in Fig. 15(a) by the cross hatching). An adhesive portion 127 is provided on an outer surface of the backsheet 123. A release sheet 128 is bonded to an outer surface of the adhesive portion 127. The pair of wings 125 are folded back on a top surface of the topsheet 122. Adhesive portions 129 are provided onto outer surfaces of the wings 125 in the folded state, respectively.

The cover sheet 130 is placed on an upper side of the pair of wings 125 and covers a surface of the napkin main body 121 on which the topsheet 122 exists. The cover sheet 130 is temporarily attached to the napkin main body 121 by means of the adhesive portions 129 provided to the pair of wings 125.

Next, brief description is given of a mounting procedure of the sanitary napkin 120. First, the release sheet 128 is peeled off from the napkin main body 121. Next, the napkin main body 121 with the cover sheet 130 attached thereto is positioned at an appropriate position of undergarment. The positioned napkin main body 121 with the cover sheet 130 attached thereto is pressed to the undergarment with the fingers or the palms. The napkin main body 121 with the cover sheet 130 attached thereto is pasted to the undergarment by means of the adhesive portion 127. Next, the cover sheet 130 is detached from the napkin main body 121. Then, the wings 125 are folded back around lateral edges of a crotch portion of the undergarment. The wings 125 thus folded are pressed to the undergarment with the fingers or the palms. The wings 125 are pasted to the undergarment by means of the adhesive portions 129.
Patent Document 1: Japanese Patent Application Publication No. Hei 8-56989
Patent Document 2: Japanese Patent Application Publication No. 2005-58783

### DISCLOSURE OF THE INVENTION

In the sanitary napkin 100 of the first conventional example, the dimensions of the cover sheet 110 in the longitudinal direction and in the width direction are larger than the dimensions of the napkin main body 101 in the longitudinal direction and in the width direction, respectively. This prevents the user from visually observing the contour of the napkin main body 101 during the positioning of the napkin main body 101 with respect to undergarment. For this reason, it is difficult to position the napkin main body 101 at an appropriate position of the undergarment.

Meanwhile, in the sanitary napkin 120 of the second conventional example, the dimensions of the napkin main body 121 in the longitudinal direction and in the width direction are smaller than the dimensions of the cover sheet 130 in the longitudinal direction and in the width direction, respectively. Accordingly, the user can visually observe the contour of the napkin main body 121 during the positioning of the napkin main body 121 with respect to undergarment. Accordingly, the sanitary napkin 120 does not have the disadvantage as in the case of the first conventional example. However, the sanitary napkin 120 of the second conventional example necessitates detaching the cover sheet 130 from the napkin main body 121 before the folding of the wings 125. For this reason, when the user pastes the wings 125 to undergarment, the fingers or the palms of the user may come into contact with the topsheet 122. Accordingly, soil, bacteria, and the like on the fingers or the palms of the user may be attached to the topsheet 122, and thus the topsheet 122 cannot be kept clean.

In this respect, an object of the present invention is to provide an absorbent article which enables an absorbent main body to be easily positioned at an appropriate position of undergarment, and which also enables a topsheet to be kept clean during the mounting process.

To achieve the above object, the invention according to claim 1, an absorbent article including an absorbent main body formed at least of a liquid-permeable topsheet configured to come into contact with a skin of a wearer and a liquid-impermeable backsheet configured to come into contact with clothing, the absorbent article includes: a cover sheet covering the topsheet, wherein the cover sheet is in contact with a contact surface of the topsheet at which the topsheet and an excretion portion of the wearer are to be in contact with each other, and both ends of the cover sheet in a longitudinal direction at least partially coincide with or recede from both ends of the absorbent main body in the longitudinal direction, and both ends of the cover sheet in a width direction at least partially coincide with or recede from both ends of the absorbent main body in the width direction.

According to claim 2, in the absorbent article according to claim 1, the absorbent main body includes a pair of wings projecting in width directions from the absorbent main body in a state of being unpacked from a packed state, and in the packed state, the pair of wings are folded back on the cover sheet.

According to claim 3, in the absorbent article according to claim 1, the cover sheet covers entirely the contact surface of the topsheet at which the top sheet and the excretion portion of the wearer are to be in contact with each other.

According to claim 4, in the absorbent article according to claim 1, an edge of the cover sheet in the longitudinal direction coincides with an edge of the absorbent main body in the longitudinal direction, and an edge of the cover sheet in the width direction coincides with an edge of the absorbent main body in the width direction.

According to claim 5, in the absorbent article according to claim 1, the cover sheet is bonded to the topsheet at a bonding position at which the topsheet and the backsheet are bonded to each other, among peripheral edge positions of the absorbent main body.

According to claim 6 in the absorbent article according to claim 1, the cover sheet contains a heat-weldable fiber.

According to claim 7, in the absorbent article according to any one of claims 1 to 6, the cover sheet has at least one of liquid-permeability and hydrophilicity.

According to claim 7, in the absorbent article according to any one of claims 1 to 6, the cover sheet has water-disintegrability.

According to the present invention, an absorbent main body can be easily positioned at an appropriate position of undergarment, and a topsheet can be kept clean during the mounting process.

### BRIEF DESCRIPTION OF THE DRAWINGS

[Fig. 1] Fig. 1 shows a first embodiment of the present invention and is an overall perspective view of a sanitary napkin.
[Fig. 2] Fig. 2 shows the first embodiment of the present invention and is a plan view of the sanitary napkin.
[Fig. 3] Fig. 3 shows the first embodiment of the present invention, and is a cross-sectional view taken along the line A-A of Fig. 2.
[Fig. 4] Fig. 4 shows the first embodiment of the present invention, and is a perspective view showing a mounting process of the sanitary napkin.
[Fig. 5] Fig. 5 shows the first embodiment of the present invention, and is a perspective view showing the mounting process of the sanitary napkin.
[Fig. 6] Fig. 6 shows a second embodiment of the present invention, and is a plan view of a sanitary napkin.
[Fig. 7] Fig. 7 shows the second embodiment of the present invention, and is a cross-sectional view taken along the line B-B of Fig. 6.
[Fig. 8] Fig. 8 shows a third embodiment of the present invention, and is a plan view of a sanitary napkin.
[Fig. 9] Fig. 9 shows the third embodiment of the present invention, and is a cross-sectional view taken along the line C-C of Fig. 8.
[Fig. 10] Fig. 10 shows a fourth embodiment of the present invention, and is a plan view of a sanitary napkin.
[Fig. 11] Fig. 11 shows the fourth embodiment of the present invention, and is a cross-sectional view taken along the line D-D of Fig. 10.
[Fig. 12] Fig. 12 shows a fifth embodiment of the present invention, and is a plan view of a sanitary napkin.
[Fig. 13] Fig. 13 shows the fifth embodiment of the present invention, and is a cross-sectional view taken along the line E-E of Fig. 12.
[Fig. 14] Fig. 14(a) is a perspective view of a sanitary napkin of a first conventional example, and Fig. 14(b) is a cross-sectional view taken along the line F-F of Fig. 14(a).
[Fig. 15] Fig. 15(a) is a perspective view of a sanitary napkin of a second conventional example, and Fig. 15(b) is a cross-sectional view taken along the line G-G of Fig. 15(a).

### BEST MODES FOR CARRYING OUT THE INVENTION

Hereinafter, embodiments of the present invention will be described on the basis of the drawings.

### (First Embodiment)

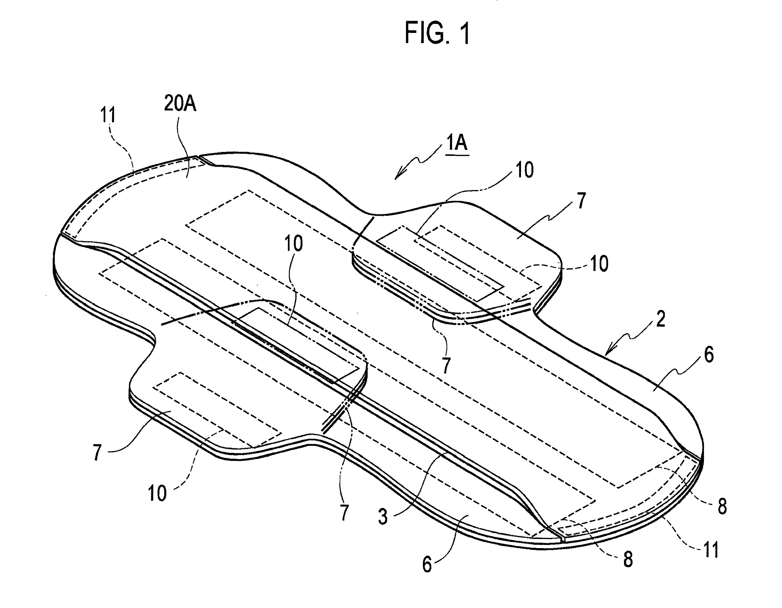

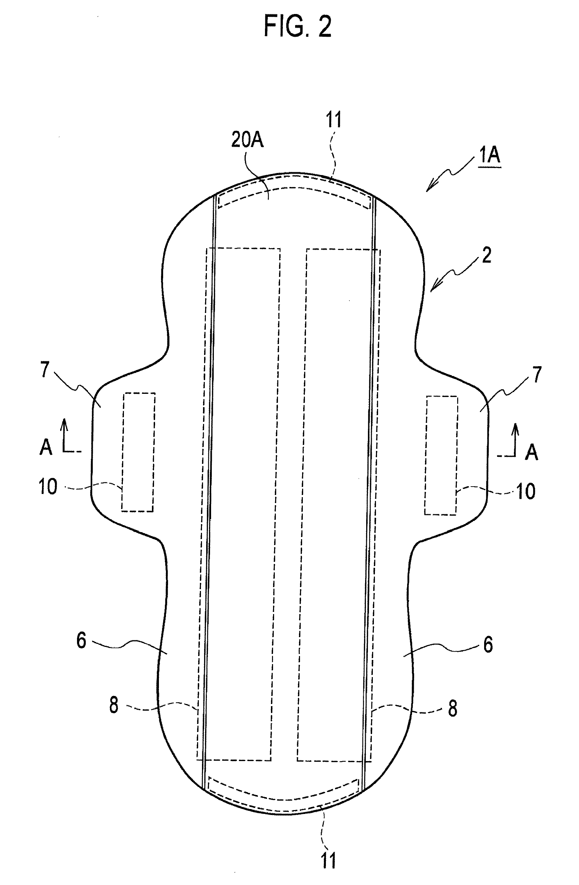

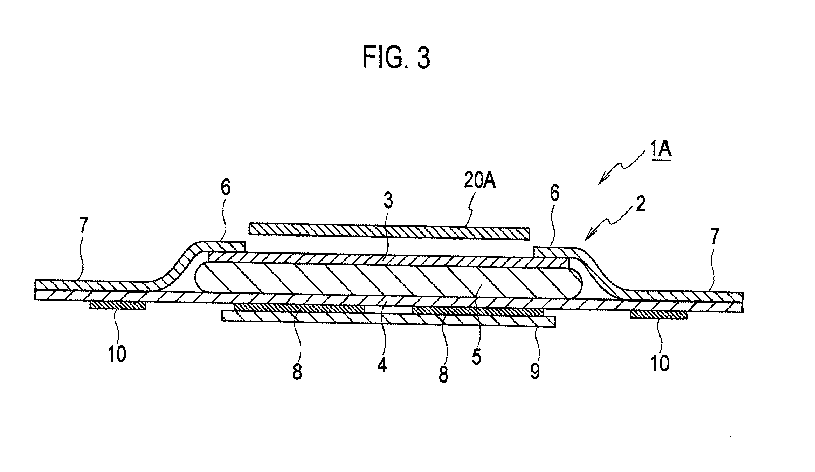

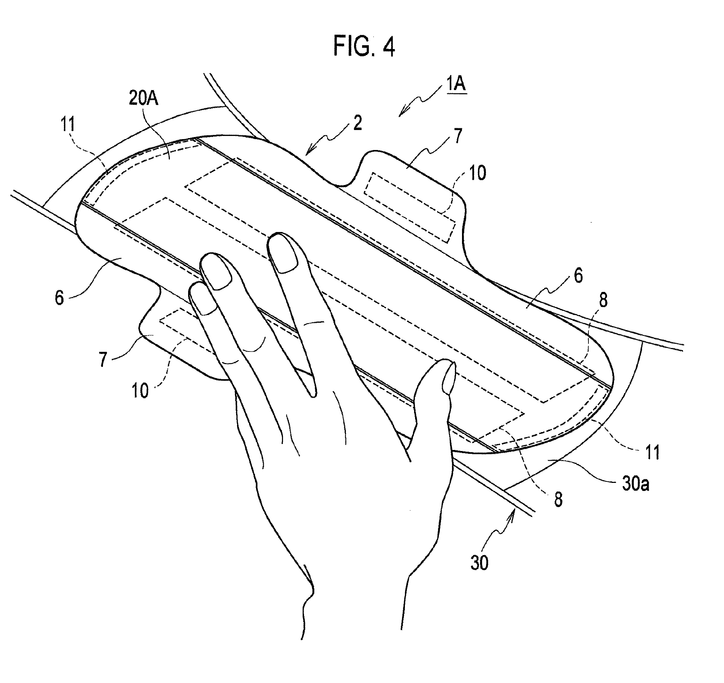

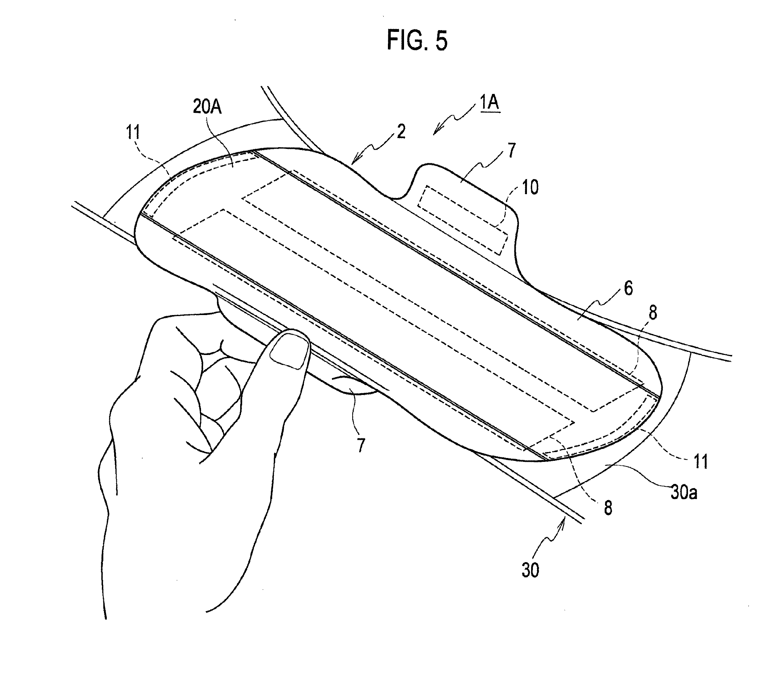

Figs. 1 to 5 show a first embodiment of the present invention. Fig. 1 is an overall perspective view of a sanitary napkin. Fig. 2 is a plan view of the sanitary napkin. Fig. 3 is a cross-sectional view taken along the line A-A of Fig. 2. Each of Figs. 4 and 5 is a perspective view showing a mounting process of the sanitary napkin.

As shown in Figs. 1 to 3, a sanitary napkin 1A, which is an absorbent article, includes a napkin main body 2, which is an absorbent main body, and a cover sheet 20A provided to the napkin main body 2.

The napkin main body 2 includes a topsheet 3, a backsheet 4, an absorber 5, a pair of side-sheets 6, and a pair of wings 7. The topsheet 3 is liquid-permeable and is configured to come into contact with the skin of a wearer. The backsheet 4 is liquid-impermeable and is configured to come into contact with undergarment, which are clothing. The absorber 5 is disposed between the topsheet 3 and the backsheet 4 and is configured to absorb liquid. The pair of side-sheets 6 are liquid-impermeable and are disposed outside outer peripheries of the topsheet 3 along the longitudinal direction. The pair of wings 7 project in width directions from outer peripheries of the pair of side-sheets 6 and of the backsheet 4 along the longitudinal direction.

The dimension of the topsheet 3 in the longitudinal direction is equal to the dimension of the backsheet 4 in the longitudinal direction. The dimension of the topsheet 3 in the width direction is smaller than the dimension of the backsheet 4 in the width direction. The dimension of the backsheet 4 in the width direction is equal to the dimension which is the total of the dimensions of the pair of side-sheets 6 in the width direction and the dimension of the topsheet 3 in the width direction. The topsheet 3 and the pair of side-sheets 6 are bonded together at mutually overlapped portions by using an adhesive paste such as a hot melt adhesive. The pair of side-sheets 6 and the backsheet 4 are bonded together by using an adhesive paste such as a hot melt adhesive over substantially the entire region of outer peripheries including outer peripheries along the longitudinal direction as well as portions projecting in the width direction. The topsheet 3 and the absorber 5 are bonded to each other at an appropriate position by using an adhesive paste such as a hot melt adhesive. Adhesive portions 8 are provided to the backsheet 4 on a contact surface thereof which is configured to come into contact with clothing. The adhesive portions 8 are configured to prevent misalignment between the napkin main body 2 and the clothing. A release sheet 9 is bonded to outer surfaces of the adhesive portions 8.

The absorber 5 includes a laminated body of crushed pulp and tissue with which the laminated body is wrapped. The absorber 5 may be an absorber obtained by blending a high-absorbent polymer powder or a thermoplastic synthetic fiber with the above-described crushed pulp.

The pair of wings 7 in a state of being unpacked from an individually packed state (shown in Fig. 1 by the solid line) project in the width direction from the napkin main body portion 2. As the pair of wings 7, portions of the pair of side-sheets 6 and portions of the backsheet 4 project in the width direction from the napkin main body portion 2. Note that the pair of wings 7 may be formed of members independent from the pair of side-sheets 6 and the backsheet 4. It is not necessary for the backsheet 4 to extend to end portions of the wings 7. Adhesive portions 10 are provided to surfaces of the pair of wings 7 on the side of the backsheet 4, respectively. The adhesive portions 10 are configured to prevent the misalignment between the napkin main body 2 and clothing. The pair of wings 7 in a before-use (individually packed) state (shown in Fig. 1 by the chain double-dashed lines) are folded back on the side of the topsheet 3. Release sheets (not shown) are bonded to outer surfaces of the adhesive potions 10.

The cover sheet 20A is formed of a material containing a heat-weldable fiber. The cover sheet 20A is disposed directly on a contact surface on which the skin of a wearer comes into contact with the topsheet 3. The pair of wings 7 are disposed on the cover sheet 20A while being in a state of being folded back on the side of the topsheet 3. The cover sheet 20A covers at least a contact surface on which the excretion portion of a wearer comes into contact with the topsheet 3. Both ends of the cover sheet 20A in the longitudinal direction and in the width direction at least partially coincide with or recede from those of the napkin main body 2 in the longitudinal direction and in the width direction. Accordingly, the contour of the napkin main body 2 can be seen by visual observation even when the topsheet 3 is covered with the cover sheet 20A. In this first embodiment, the dimensions of the cover sheet 20A in the longitudinal direction and in the width direction are substantially equal to the dimensions of the topsheet 3 in the longitudinal direction and in the width direction, respectively. The positions of front and rear edges of the cover sheet 20A in the longitudinal direction are the same as the positions of front and rear edges of the napkin main body 2 in the longitudinal direction. The cover sheet 20A covers entirely the contact surface of the topsheet 3 at which the topsheet 3 and the skin of a wearer are to be in contact with each other.

The cover sheet 20A is temporarily attached to the topsheet 3 at the same positions as the bonding positions at which the topsheet 3 and the backsheet 4 are bonded to each other by using an adhesive paste such as a hot melt adhesive. Specifically, the cover sheet 20A is temporarily attached to the topsheet 3 at a front end position and a rear end position among peripheral edge positions of the napkin main body 2 by means of heat-welding portions 11.

Conventionally, in a manufacturing process of an absorbent article, the absorber 5 is shaped, and then the topsheet 3 and the backsheet 4 are bonded to each other by using an adhesive paste such as a hot melt adhesive with the absorber 5 interposed therebetween. After the topsheet 3 and the backsheet 4 are bonded to each other, the topsheet 3 and the backsheet 4 are welded to each other by heating outer peripheries of the absorber 5 over certain widths, for the purpose of further increasing the bonding strength of the bonded portions. This results in the fixation (sealing) of the absorber 5 at a predetermined position. After the sealing, the absorber 5 is cut into a shape of a product. Meanwhile, in a manufacturing process of this embodiment additionally includes a step of folding the cover sheet 20A to be on the contact surface of the topsheet 3, the skin of a wearer coming into contact with the contact surface, thereby fixing the absorber at a predetermined position. The step of folding is included before the sealing step of welding an outer peripheral portion of the absorber 5. A sealing step is performed with the cover sheet 20A, the topsheet 3, and the backsheet 4 stacked together. When the cover sheet 20A or the topsheet 3 contains a heat-weldable fiber, pieces of the heat-weldable fiber are thermally welded to each other by heat. As a result, the cover sheet 20A and the topsheet 3 are temporarily attached to each other, without providing a paste or an adhesive between the cover sheet 20A and the topsheet 3. Accordingly, the cover sheet 20A can be temporarily attached without adding a separate temporarily attaching step of the cover sheet 20A, thereby preventing the manufacturing process from being complicated.

The cover sheet 20A is formed of a flexible and hydrophilic sheet member. Specifically, non-woven fabrics (such as an air-through non-woven fabric, a spunbond non-woven fabric, a point bond non-woven fabric, and a spunlace non-woven fabric) and airlaid pulp sheets are preferable. Among those a spunlace non-woven fabric is particularly preferable. The spunlace non-woven fabric is a non-woven fabric manufactured by the spunlace method from a substantially uniform blend of 60% of a rayon fiber of 1.7 dtex with a fiber length of 40 mm and 40% of a polyester fiber of 2.2 dtex with a fiber length of 51 mm. The cover sheet 20A has a weight per square meter (hereinafter referred to as a basis weight) of 50 g/m2, and a thickness of 0.55 mm.

In the sanitary napkin 1A configured as above, the napkin main body 2 in the before-use state is folded along with the cover sheet 20A, and housed in an individual package, in general.

Next, brief description is given of a mounting procedure of the sanitary napkin 1A. First, undergarment 30 are displaced to a position around the knees. If being in an individually packed state, the individual package is peeled off, and the sanitary napkin 1A is taken out. Then the sanitary napkin 1A is unfolded. When the individual package is peeled off, the release sheets 9 (not shown) provided to the backsheet 4 and the pair of wings 7 of the sanitary napkin 1A are peeled off. Next, the pair of wings 7 are held by the hand, and the napkin main body 2 is positioned at an appropriate position of the undergarment 30. As shown in Fig. 4, the positioned napkin main body 2 is pressed to the undergarment 30 with the fingers or the palms to thereby paste the napkin main body 2 to the undergarment 30 by means of the adhesive portions 8. Next, as shown in Fig. 5, the wings 7 are folded back at lateral edges of a crotch portion 30a of the undergarment 30. The folded-back wings 7 are pressed to the undergarment 30 with the fingers or the palms, thereby pasting the wings 7 to the undergarment 30 by means of the adhesive portions 10. Thereafter, the cover sheet 20A is peeled off from the napkin main body 2. This completes the mounting. Note that, the adhesive portions 8 provided to the backsheet 4 may be pasted to the undergarment 30 after the adhesive portions 10 of the wings 7 are pasted to the undergarment 30.

As has been described above, in the sanitary napkin 1A, the cover sheet 20A, which is detachable, is provided on the surface of the topsheet 3 of the napkin main body 2. The cover sheet 20A covers at least the contact surface on which the excretion portion of the wearer comes into contact with the topsheet 3. The length of the cover sheet 20A in the longitudinal direction is equal to the length of the napkin main body 2 in the longitudinal direction. The cover sheet 20A is disposed directly on the contact surface on which the skin of the wearer comes into contact with the topsheet 3. Accordingly, the user can visually observe the contour of the napkin main body 2 when mounting the napkin main body 2 on the undergarment 30. This enables the napkin main body 2 to be easily mounted on the undergarment 30 at an appropriate position. Moreover, since the napkin main body 2 with the cover sheet 20A attached thereto can be mounted on the undergarment 30, the user can perform the mounting without direct contact with the topsheet 3. Then, the cover sheet 20A can be detached from the napkin main body 2 after the napkin main body 2 is mounted on the undergarment 30. Accordingly, the topsheet 3 is kept clean during the mounting process.

The cover sheet 20A is hydrophilic. Accordingly, the cover sheet 20A detached from the napkin main body 2 can be used for wiping, and hence is convenient. Especially, the cover sheet 20A of this embodiment is formed of a spunlace non-woven fabric. The basis weight of the cover sheet 20A is 50 g/m2, and the thickness thereof is 0.55 mm. Since this non-woven fabric is relatively thick, sufficient wiping can be achieved even when the skin is wet.

The cover sheet 20A has an outer diameter size enough to cover entirely the contact surface of the topsheet 3 at which the topsheet 3 and the skin of the wearer are to be in contact with each other. Accordingly, the fingers of the user can be surely prevented from coming into contact with the topsheet 3.

The cover sheet 20A contains a heat-weldable fiber. Moreover, as described above, the cover sheet 20A is stacked on the contact surface on which the skin of the wearer comes into contact with the topsheet 3, and then the sealing step is performed. In the sealing step, the absorber 5 is fixed between the topsheet 3 and the backsheet 4, and then cut into a shape of a product. Accordingly, the cover sheet 20A is temporarily attached to the topsheet 3 at the same positions as the positions at which the topsheet 3 and the backsheet 4 are bonded to each other, among peripheral edge positions of the napkin main body 2. Here, since no adhesive paste or the like is provided between the cover sheet 20A and the napkin main body 2, the wearer can easily peel off the cover sheet 20A when mounting the napkin. Accordingly, this attachment is referred to as "temporary attachment."

As deceived above, without providing a separate temporarily attaching step for the temporary attachment of the cover sheet 20A, the cover sheet 20A can be temporarily attached to the topsheet 3 by means of the heat-welding portions 11. Accordingly, the manufacturing process can be prevented from being complicated. Moreover, in the topsheet 3, no temporary-attachment portions exists in a central region encompassing at least the contact surface with which the excretion portion of the wearer comes into contact. Accordingly, no damage due to the temporary attachment is inflicted on the topsheet 3, and a favorable texture can surely be obtained in at least the central region of the topsheet 3. Furthermore, partial exposure of the topsheet 3 does not occur, which would otherwise occur when the cover sheet 20A is partially curled up. Accordingly, the topsheet 3 can surely be protected. Moreover, the wearer can easily detach the cover sheet 20A from the napkin main body 2 by pinching a center portion of the cover sheet 20A. Furthermore, since the cover sheet 20A is temporarily attached to the napkin main body 2 by means of the heat-welding portions 11, no paste or adhesive remains at positions at which the cover sheet 20A is bonded, after the cover sheet 20A is detached. Accordingly, not only the topsheet 3 can be kept clean, but also the deterioration in texture can be prevented in the central region encompassing the contact surface with which the excretion portion of the wearer come into contact.

The sanitary napkin 1A has the pair of wings 7. When the pair of wings 7 are folded back on the side of the topsheet 3, the pair of wings 7 are disposed on a top surface of the cover sheet 20A. The wearer can mount the wings 7 on the lateral edges of the crotch portion 30a of the undergarment 30 with the cover sheet 20A being attached to the napkin main body 2. Accordingly, in the mounting process, the topsheet 3 can be kept clean. The present invention can of course be applied to sanitary napkins having no wings.

Note that, as in the case of this embodiment, when the sanitary napkin 1A having the wings 7 is produced, the topsheet 3 and the side-sheets 6 constituting the wings 7 are integrated, and then the topsheet 3, the wings 7, and the backsheet 4 are joined together by using a paste or a adhesive. Next, the cover sheet 20A is stacked on the contact surface on which the skin of the wearer comes into contact with the napkin main body 2 and then the topsheet 3 and the backsheet 4 are sealed. Preferably, after the sealing, cutting into a shape of a product is performed, and then the wings 7 are folded back to be on the top surface of the cover sheet 20A stacked on the contact surface on which the skin of the wearer comes into contact with the napkin main body 2.

### (Second Embodiment)

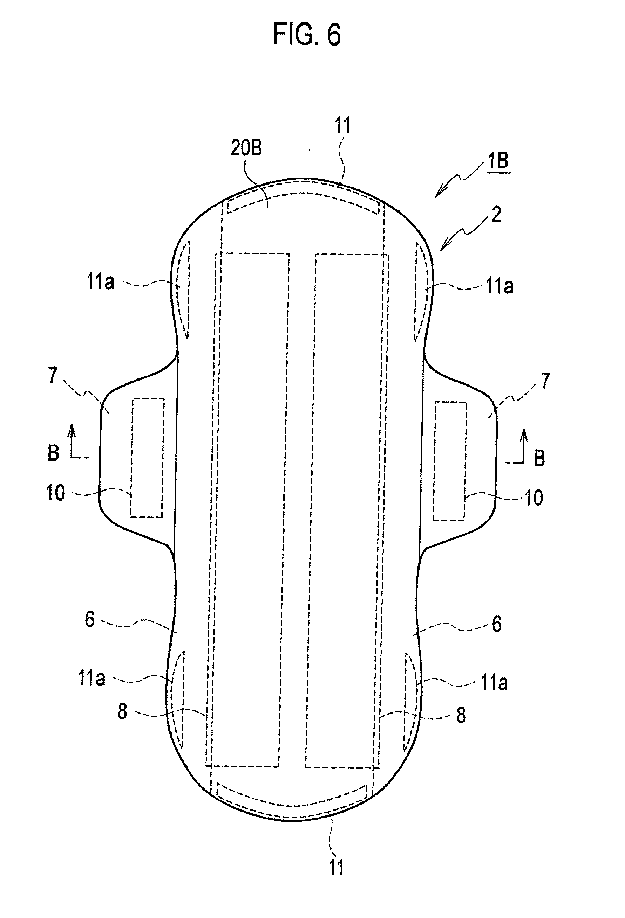

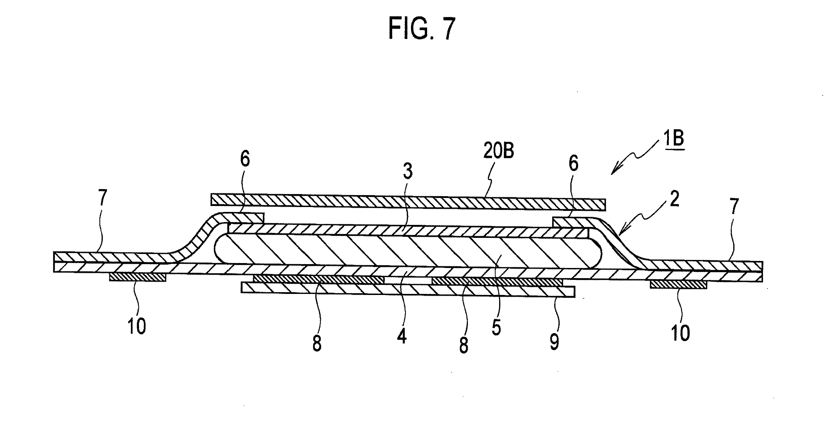

Figs. 6 and 7 show a second embodiment of the present invention. Fig. 6 is a plan view of a sanitary napkin, and Fig. 7 is a cross-sectional view taken along the line B-B of Fig. 6.

When a sanitary napkin 1B according to this second embodiment is compared with the sanitary napkin 1A according to the above-described first embodiment, the sanitary napkin 1B has a cover sheet 20B which has a different structure and a different temporarily attaching mechanism thereof.

Specifically, as shown in Figs. 6 and 7, the cover sheet 20B has a contour size which is substantially equal to that of the napkin main body 2 excluding the pair of wings 7. In addition, the cover sheet 20B is temporarily attached at the front and rear positions and at four corner positions on both sides among the peripheral edge positions of the napkin main body 2, by means of the heat-welding portions 11, and heat-welding portions 11a.

Other components are the same as those in the first embodiment, and hence description thereof is omitted. In the drawings, same reference signs denote same components for clarification.

According to the second embodiment, the entire region of the contact surface (the topsheet 3 and the side-sheets 6) on which the skin of the wearer comes into contact with the napkin main body 2 can be kept clean.

### (Third Embodiment)

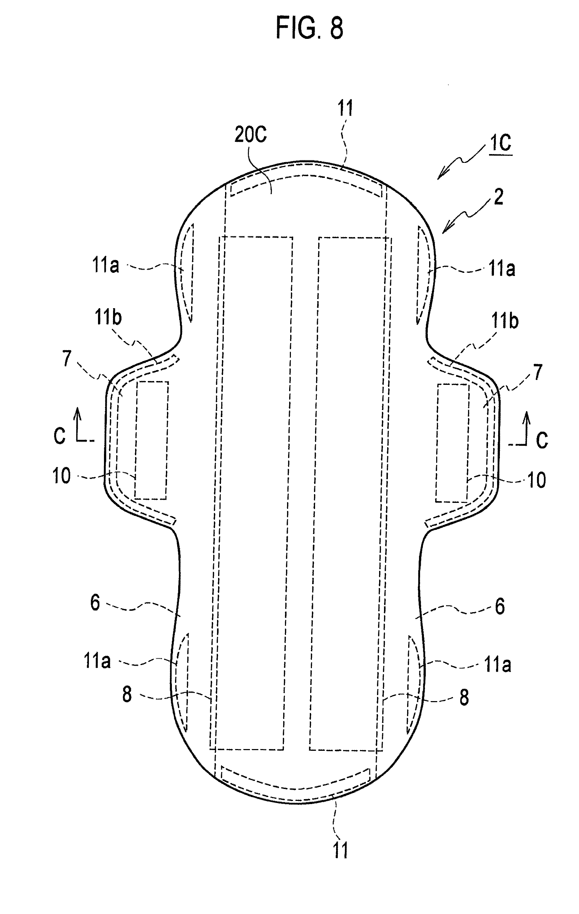

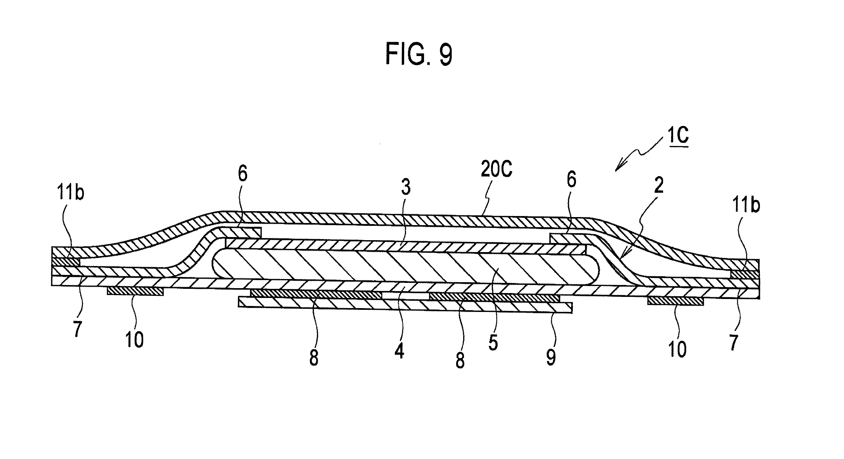

Figs. 8 and 9 show a third embodiment of the present invention. Fig. 8 is a plan view of a sanitary napkin, and Fig. 9 is a cross-sectional view taken along the line C-C of Fig. 8.

When a sanitary napkin 1C according to this third embodiment is compared with the sanitary napkin 1A according to the above-described first embodiment, the sanitary napkin 1C has a cover sheet 20C which has a different structure and a different temporarily attaching mechanism thereof.

Specifically, as shown in Figs. 8 and 9, the cover sheet 20C has a contour size which is substantially equal to that of the napkin main body 2 excluding the pair of wings 7. In addition, the cover sheet 20C is temporarily attached at the front and rear positions, at four corner positions on both sides, and at peripheral edge positions of the wings 7 among the peripheral edge positions of the napkin main body 2, by means of the heat-welding portions 11, the heat-welding portions 11a, and heat-welding portions 11b.

Other components are the same as those in the first embodiment, and hence description thereof is omitted. In the drawings, same reference signs denote same components for clarification.

According to the third embodiment, the entire region of the contact surface (the topsheet 3 and the side-sheets 6) on which the skin of the wearer comes into contact with the napkin main body 2 can be kept clean. Moreover, the productivity is excellent because, in the sealing step during the manufacturing, the cover sheet 20C is temporarily attached to the napkin main body 2 by means of the heat-welding portions 11, 11a, and 11b, and then the temporarily attached cover sheet 20C is cut into the same size as that of the napkin main body 2. Moreover, the wings 7 can be kept clean.

### (Fourth Embodiment)

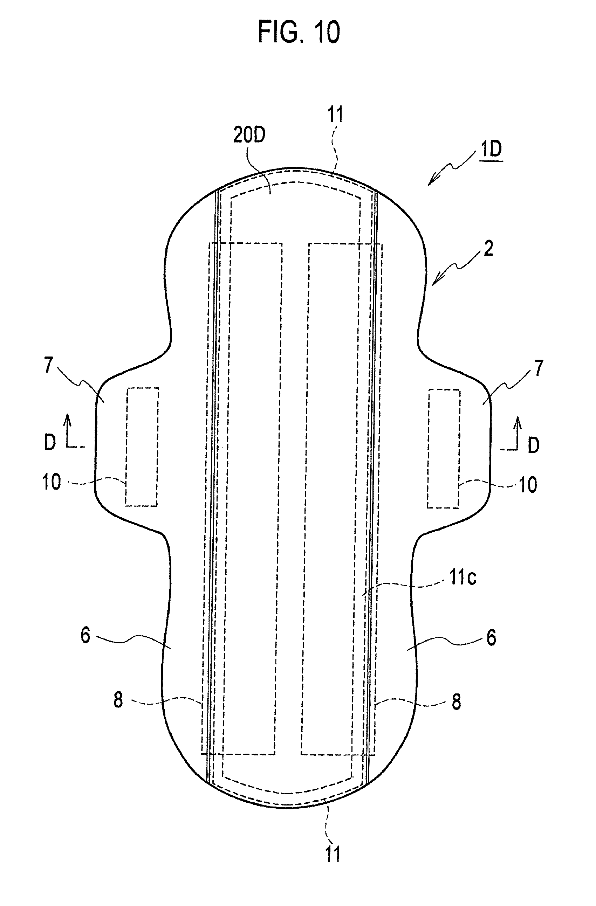

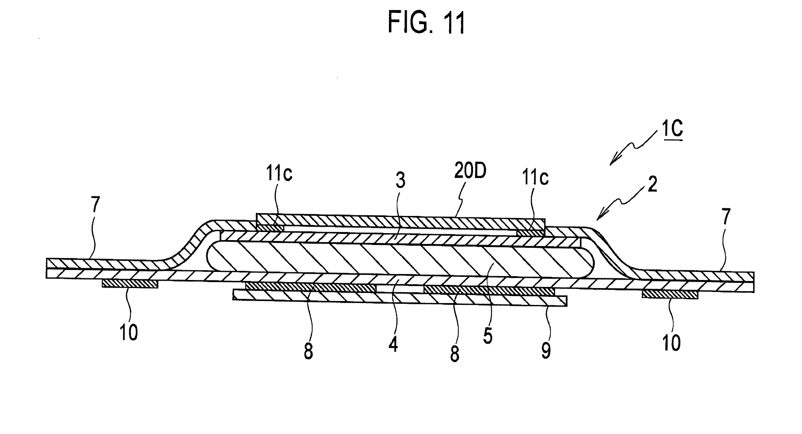

Figs. 10 and 11 show a fourth embodiment of the present invention. Fig. 10 is a plan view of a sanitary napkin, and Fig. 11 is a cross-sectional view taken along the line D-D of Fig. 10.

When a sanitary napkin 1D according to this fourth embodiment is compared with the sanitary napkin 1A according to the above-described first embodiment, the sanitary napkin 1D is different only in the temporarily attaching mechanism of a cover sheet 20D.

Specifically, as shown in Figs. 10 and 11, the cover sheet 20D has the same contour size as that of the topsheet 3, as in the case of the first embodiment. The entire peripheral edge of the cover sheet 20D is temporarily attached to the topsheet 3 by means of the heat-welding portions 11, as well as and heat-welding portions 11c.

Other components are the same as those in the first embodiment, and hence description thereof is omitted. In the drawings, same reference signs denote same components for clarification.

According to the fourth embodiment, unless the cover sheet 20D is peeled off, the cleanness of the topsheet 3 can be secured almost completely.

### (Fifth Embodiment)

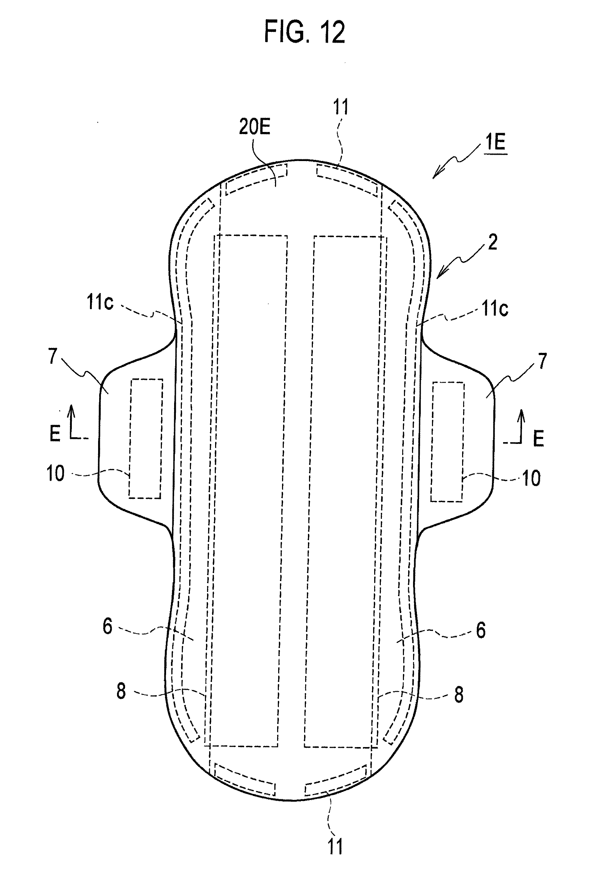

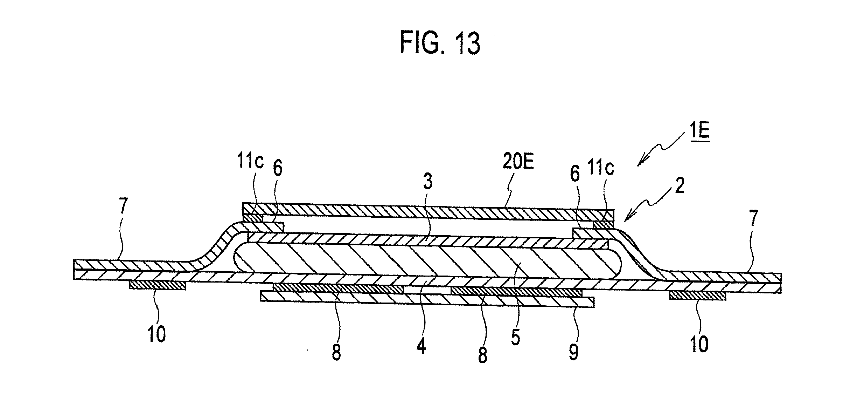

Figs. 12 and 13 show a fifth embodiment of the present invention. Fig. 12 is a plan view of a sanitary napkin, and Fig. 13 is a cross-sectional view taken along the line E-E of Fig. 12.

When a sanitary napkin 1E according to this fifth embodiment is compared with the sanitary napkin 1B according to the above-described second embodiment, the sanitary napkin 1E is different only in the temporarily attaching mechanism/structure of a cover sheet 20E.

Specifically, as shown in Figs. 12 and 13, the cover sheet 20E has substantially the same contour size as that of the napkin main body 2 excluding the pair of wings 7. In addition, the cover sheet 20E is temporarily attached at the front and rear positions and at all lateral positions on the both sides among peripheral edge positions of the napkin main body 2 by means of the heat-welding portions 11, 11c. The heat-welding portion 11 at the front position is divided into pieces at two positions, and the napkin main body 2 is not temporarily attached at a front center position.

Other components are the same as those in the first embodiment, and hence description thereof is omitted. In the drawings, same reference signs denote same components for clarification.

According to the fifth embodiment, the entire contact surface of the topsheet 3 and the side-sheets 6 can be kept clean, the skin of the wearer coming into contact with the napkin main body 2 on the contact surface. Moreover, since the temporarily attaching portion is divided at the front position of the cover sheet 20E, the fifth embodiment has an advantage that the cover sheet 20E can easily peeled off. Specifically, the cover sheet 20E can also be peeled off by pinching the center of the cover sheet 20E at the front position with the fingers. Note that, the heat-welding portion 11 of the cover sheet 20E at the rear position may be divided, or both the heat-welding portions 11 at the front position and at the rear position may be divided.

### (Dimensions of Cover sheet)

The above-described embodiments have shown the cases where the cover sheets 20A to 20E have various contour dimensions. However, each of the cover sheets only needs to cover at least the contact surface on which the excretion portion of the wearer comes into contact with the topsheet 3. It is only necessary that the both ends of the cover sheet in the longitudinal direction and in the width direction at least partially coincide with or recede from the both ends of the napkin main body 2, respectively. With such contour dimensions, the wearer can see the contour of the napkin main body 2 through a visual observation from above the cover sheet. Accordingly, the wearer can easily position the napkin main body 2 at an appropriate position of undergarment. Moreover, since the topsheet 3 is covered with the cover sheet, the topsheet 3 can be kept clean in the mounting process.

### (Modifications of cover sheet and the like)

When the cover sheets 20A to 20E are used for wiping, the basis weight of each of the cover sheets 20A to 20E is preferably 20 to 120 g/m², and further more preferably 30 to 80 g/m². If the basis weight is less than 20 g/m², substances wiped by the cover sheets 20A to 20E may seep out of the cover sheets 20A to 20E, and soil the hand of the wearer at the wiping of the body. If the basis weight exceeds 120 g/m², the cover sheet is too hard for use in wiping the body. Moreover, besides the basis weight, the bending resistance determined by the cantilever method is preferably 100 mm or less, and further preferably 80 mm or less. If the bending resistance exceeds 100 mm, the drape property becomes poor, and the wiping performance is also lowered.

Note that, when each of the cover sheets 20A to 20E is not intended to be used for wiping but intended to be used only for covering the topsheet 3, each of the cover sheets 20A to 20E may be a film, and in such a case a basis weight of 10 g/m² or more is sufficient.

Each of the cover sheets 20A to 20E may be formed of a liquid-permeable material. Each of the liquid-permeable cover sheets 20A to 20E are formed, for example, by subjecting a hydrophobic fiber to a hydrophilic treatment. With the use of a liquid-permeable sheet member, menstrual blood and the like can permeate into the underlining layers even when the wearer conducts the mounting without peeling the cover sheet 20A by mistake.

Each of the cover sheets 20A to 20E may be formed of a water-disintegrable material. Specifically, the water-disintegrable material is one obtained by substantially uniformly blending 70% of pulp, 25% of rayon of 1.1 dtex with a fiber length of 7 mm, and 5% of Tencel of 1.7 dtex with a fiber length of 3 mm with each other, and then forming a sheet therefrom. The basis weight is 45 g/m², and the thickness is 0.5 mm. The use of the water-disintegrable material allows the cover sheets 20A to 20E to be flushed down a toilet, and hence provides convenience.

Each of the cover sheets 20A to 20E may be formed of an antibacterial material. A specific example is a spunlace non-woven fabric containing a catechin component. It is possible to inhibit proliferation of unwanted bacteria at the excretion portion of the wearer.

Each of the cover sheets 20A to 20E may be formed of a sheet member having a deodorizing capability. This can prevent the formation of odor. More preferably, each of the cover sheets 20A to 20E is provided with two or more functionalities by combining the above-described various materials. For example, as the cover sheets 20A to 20E, those which have both liquid-permeability and hydrophilicity are used. The use of such sheets improves the wiping performance of the cover sheets 20A to 20E in the use of wiping. Alternatively, as the cover sheets 20A to 20E, those which have both liquid-permeability and water-disintegrability are used. The use of such sheets enables the cover sheets 20A to 20E to be flushed down a toilet after the use for wiping, providing an extremely high convenience.

A moisture retentive material may be applied to the cover sheets 20A to 20E. Moreover, the cover sheets 20A to 20E may be passed through a liquid agent in which a functional material is dissolved. Alternatively, a portable spray can filled with a liquid agent in which a functional material is dissolved is included in a package of a product of the sanitary napkin 1A, or is sold separately therefrom. The wearer can provide the cover sheets 20A to 20E with desired functionalities by spraying the liquid agent in which the functional material is dissolved onto the cover sheets 20A to 20E, as needed.

The 5% elongation strength of each of the cover sheets 20A to 20E in a conveyance direction at the time of manufacturing is preferably 0.2 N/25 mm or higher because of the following reason. Specifically, if the 5% elongation strength is less than 0.2 N/25 mm, the cover sheets 20A to 20E are elongated in the conveyance direction in the manufacturing at the time of the manufacturing and conveying. If the cover sheets 20A to 20E are temporarily attached while being stretched, a stress to restore the original state acts on the cover sheets 20A to 20E, and hence the temporarily attached cover sheets 20A to 20E may be peeled off before use.

Each of the cover sheets 20A to 20E may be temporarily attached to the napkin main body 2 while being folded one or more times. When each of the cover sheets 20A to 20E peeled off from the napkin main body 2 is unfolded, the usable area is enlarged. Accordingly, such cover sheets 20A to 20E are convenient.

### (Absorbent Article other than Sanitary Napkin)

The above-described embodiments have shown the cases where the present invention is applied to the sanitary napkins 1A to 1E, which are absorbent articles. However, the present invention is applicable to so-called liners, incontinence aids (also referred to as incontinence pads) and the like. Examples of the liners, and incontinence pads to which the present invention is applicable include those of a type in which a topsheet and a backsheet are provided but no absorber is provided between the topsheet and the backsheet, those of a type in which a topsheet, an absorber, and a backsheet are provided, and the like.

In many cases, the conveyance direction in the manufacturing of the sanitary napkins 1A to 1E is a longitudinal direction of the products. However, unlike the conveyance direction in the manufacturing of the sanitary napkins 1A to 1E, the conveyance direction in the manufacturing of liners is the width direction of the products in many cases. Accordingly, in the cases of liners, an edge of each cover sheet in the longitudinal direction can be easily trued up with an edge of each napkin main body, which is an absorbent main body, in the longitudinal direction. Specifically, the cutting into the cover sheets and the cutting into the absorbent main bodies can be performed in a single cutting step.

### (Others)

Each of the sanitary napkins 1A to 1E is preferably provided with a notice prohibiting the use thereof with each of the cover sheets 20A to 20E attached to the napkin main body 2. However, if the cover sheets 20A to 20E are made of a liquid-permeable material, the body fluid can permeate to the side of the napkin main body 2 though the cover sheets 20A to 20E, and the original functionalities of the sanitary napkins 1A to 1E can be retained, even when the napkin main body 2 is mounted with the cover sheets 20A to 20E remaining thereon.

Note that the entire contents of Japanese Patent Application No. 2008-016469 (filed on January 28, 2008) are incorporated herein by reference.

### INDUSTRIAL APPLICABILITY

As has been described above, the absorbent article according to the present invention enables the absorbent main body to be easily positioned at an appropriate position of undergarment, and enables the topsheet to be kept clean during the mounting process. Accordingly, the absorbent article is useful for liners, incontinence aids, diapers, and sanitary napkins.

## Claims

1. An absorbent article including an absorbent main body formed at least of a liquid-permeable topsheet configured to come into contact with a skin of a wearer and a liquid-impermeable backsheet configured to come into contact with clothing, the absorbent article comprising:
a cover sheet covering the topsheet, wherein
the cover sheet is in contact with a contact surface of the topsheet at which the topsheet and an excretion portion of the wearer are to be in contact with each other, and
both ends of the cover sheet in a longitudinal direction at least partially coincide with or recede from both ends of the absorbent main body in the longitudinal direction, and
both ends of the cover sheet in a width direction at least partially coincide with or recede from both ends of the absorbent main body in the width direction.

2. The absorbent article according to claim 1, wherein
the absorbent main body includes a pair of wings projecting in width directions from the absorbent main body in a state of being unpacked from a packed state, and
in the packed state, the pair of wings are folded back on the cover sheet.

3. The absorbent article according to claim 1, wherein the cover sheet covers entirely the contact surface of the topsheet at which the top sheet and the excretion portion of the wearer are to be in contact with each other.

4. The absorbent article according to claim 1, wherein
an edge of the cover sheet in the longitudinal direction coincides with an edge of the absorbent main body in the longitudinal direction, and
an edge of the cover sheet in the width direction coincides with an edge of the absorbent main body in the width direction.

5. The absorbent article according to claim 1, wherein the cover sheet is bonded to the topsheet at a bonding position at which the topsheet and the backsheet are bonded to each other, among peripheral edge positions of the absorbent main body.

6. The absorbent article according to claim 1, wherein the cover sheet contains a heat-weldable fiber.

7. The absorbent article according to claim 1, wherein the cover sheet has at least one of liquid-permeability and hydrophilicity.

8. The absorbent article according to claim 1, wherein the cover sheet has water-disintegrability.
